# EUROPEAN PATENT SPECIFICATION

(11) **EP 2 427 473 B1**
(45) Date of publication and mention of the grant of the patent: **03.04.2013**
(21) Application number: 10727869.9
(22) Date of filing: 06.05.2010
(51) Int. Cl.: C07J 9/00, C07J 41/00

(54) **METHOD FOR THE SYNTHESIS OF GLYCOCHOLIC ACID**
VERFAHREN ZUR SYNTHESE VON GLYCOCHOLSÄURE
PROCÉDÉ POUR LA SYNTHÈSE D'ACIDE GLYCOCHOLIQUE

(30) Priority: 08.05.2009 IT MI20090782
(43) Date of publication of application: 14.03.2012
(73) Proprietor: PRODOTTI CHIMICI E ALIMENTARI SPA, 15060 Basaluzzo (Alessandria) (IT)
(72) Inventor: GALDI, Gianluca, I-16030 Pieve Ligure (Genova) (IT); PARENTI, Massimo, I-15067 Novi Ligure (Alessandria) (IT); ZUCCOTTI, Pierfranca, I-15068 Pozzolo Formigaro (Alessandria) (IT)
(74) Representative: Pistolesi, Roberto
(86) International application number: PCT/IB2010/051990
(87) International publication number: WO 2010/128472

(56) References cited:
- IT-B- 1 239 952
- CURRAGH E F ET AL: "Kinetics and mechanism of catalysis by proteolytic enzymes. 2. Kinetic studies of thrombin-catalysed reactions and their modification by bile salts and other detergents." October 1964 (1964-10), THE BIOCHEMICAL JOURNAL OCT 1964, VOL. 93, NR. 1, PAGE(S) 163 - 171 , XP002573853 ISSN: 0264-6021 page 164, column 2, paragraph 4
- S. BERGSTRÖM ET AL: ACTA CHEMICA SCANDINAVICA., vol. 7, 1953, pages 1126-1127, XP002573854 MUNKSGAARD, COPENHAGEN. ISSN: 0904-213X
- WILLEMEN, HENDRA M. ET AL: "Aggregation of different amino acid conjugates of cholic acid in aqueous solution" COLLOIDS AND SURFACES, A: PHYSICOCHEMICAL AND ENGINEERING ASPECTS , 218(1-3), 59-64 CODEN: CPEAEH; ISSN: 0927-7757, 2003, XP002573855

## Description

The present invention regards a new process for the synthesis of glycocholic acid

### PRIOR ART

Glycocholic acid is a biliary salt involved in the formation of mixed micelles for the absorption of fats. It is present as a sodium salt in the bile of mammals.

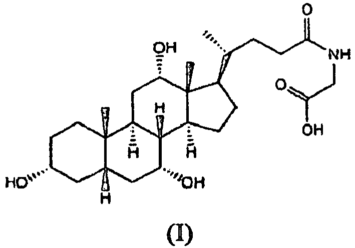

### Glycocholic acid

Glycocholic acid is generally prepared through the conjugation of cholic acid with glycine, through an amide bond.

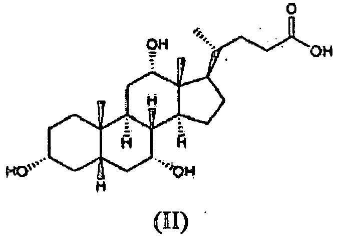

### Cholic acid

One of the simplest methods for attaining an amide bond is the acylation of an amine, or another nucleophile, with the anhydrides of the carboxylic acids, both symmetric and asymmetric. Biochem. J. vol. 93, 163-71 (1964) discloses the production of glycocholic acid via a mixed anhydride reacted with C-protected glycine in DMF/dioxan.

Generally, in the replacement reaction between the anion of an amino acid and an asymmetric anhydride, a less reactive anhydride is formed, with the elimination of the anion corresponding to the stronger acid (see Corby, Kenner and Todd, J. Chem. Soc. (1952), page 1234 and Organic Reactions, Volume 12, page 163-352, John Wiley & Son, New York (1962)).

In the reaction between an anhydride and an amine, the amine behaves like a nucleophile and the acylating species like an electrophile reagent. The effect of the solvent during the peptide synthesis is very important (see: Elkik and Gault in Compt rend. 238, 2428 (1954) and Emery and Gold, J. Chem. Soc. (1950), 1443, 1447, 1455).

Generally, in aqueous media the reaction for forming the amide bond reduces drastically.

In the synthesis of glycocholic acid through the method of the mixed anhydride described above, generally the cholic acid and the glycine hydrochloride ethyl-ester are dissolved in solvents such as dimethylformamide (DMF) or dioxan; then the base of the glycine hydrochloride ethyl-ester is freed and the carboxylic group of cholic acid is salified with an organic base, still in the same solvents, i.e. DMF or dioxan.

Through addition onto the cholic acid salt of a chloroformate, there occurs the formation of the mixed anhydride and ensuing precipitation of the hydrochloride base.

After eliminating the hydrochloride base through filtration, there occurs the acylation of glycine ethyl-ester with the mixed synthesised anhydride, thus attaining the conjugated biliary acid.

Given the interest for glycocholic acid, there arises the need for alternative methods of synthesis capable of providing this product with high yields and a high degree of purity.

### SUMMARY OF THE INVENTION

Thus, an object of the present invention is a process for preparing glycocholic acid of formula (I) comprising the following steps: a) making a mixed anhydride of cholic acid through the suspension of cholic acid in acetone or aqueous acetone in presence of a base, and subsequent reaction with an alkyl chloroformate; b) aminolysis of the so obtained mixed anhydride of cholic acid with an aqueous solution of a glycine ester, resulting in an ester of glycocholic acid; c) hydrolysis of the so obtained ester of glycocholic acid through the treatment with a strong aqueous base and precipitation of glycocholic acid through acidification of the corresponding sodium salt.

### DESCRIPTION OF THE INVENTION

The object of the present invention is an alternative method for the synthesis of glycocholic acid comprising the following steps:
a) formation of a mixed anhydride of cholic acid through the suspension of cholic acid in acetone or in aqueous acetone in presence of a base, and subsequent reaction with an alkyl chloroformate; b) aminolysis of the so obtained mixed anhydride of cholic acid with an aqueous solution of a glycine ester, resulting in an ester of glycocholic acid.

The process subject of the present invention may optionally comprise the following step:
c) hydrolysis of the ester of glycocholic acid obtained in step b) through the treatment with a strong aqueous base and precipitation of glycocholic acid through acidification of the corresponding sodium salt.

Performed in the reaction step a) is the suspension of cholic acid in acetone or in aqueous acetone, in presence of a base, at ambient temperature, i.e. comprised between 18 and 25 °C. The base is preferably an amine, even more preferably a trialkylamine and even more preferably triethylamine or tributylamine.

Added to the suspension thus obtained, cooled at a temperature between -5 °C and +10 °C, preferably between 0 °C and +5°C, is an alkyl chloroformate, preferably methyl chloroformate, ethyl chloroformate and propyl chloroformate, and even more preferably ethyl chloroformate, maintaining the suspension cooled as above, thus obtaining a suspension of the mixed anhydride of cholic acid and of the hydrochloride of the base used.

Thus, it has been surprisingly discovered that by using acetone or aqueous acetone as a reaction medium for the synthesis of the mixed anhydride of cholic acid and water as the solvent for preparing the amine, it is possible to avoid the filtering step of the previous syntheses, required for the removal of hydrochloride amine that forms during the synthesis of the mixed anhydride.

As a matter of fact, the suspension obtained in step a) may be directly used in the reaction step b), wherein the mixed anhydride of cholic acid is aminolysed with an aqueous solution of a glycine ester, preferably C₁-C₄ alkyl ester, even more preferably glycine ethyl ester.

The aqueous solution of glycine ester is mixed with the suspension of the mixed anhydride of cholic acid; the mixture thus obtained is stirred, preferably lasting for a time comprised between about 10 and 14 hours, preferably about 12 hours. The biliary acid that is formed through hydrolysis of the mixed anhydride with the aqueous solution of the ester of glycine mainly remains dissolved in the hydroacetonic solution, while the conjugated biliary acid, i.e. cholic acid conjugated with glycine ester, precipitates over time in crystalline form. Optionally, executed may be a further step of purifying the cholic acid conjugated with glycine ester, through crystallization thereof in aqueous acetone, obtaining a particularly pure product, with an amount of free biliary acid much lower than 0.5%, with respect to the amount of conjugated biliary acid.

Provided for in the optional stage c) is the hydrolysis of the conjugated biliary acid esterified with a strong aqueous base, such as sodium hydroxide (NaOH) or potassium hydroxide (KOH).

The possible ester of the conjugated biliary acid still present after the hydrolysis may be eliminated by washing using acetate ethyl at controlled pH, between 6.5 and 7.5. Lastly, obtained is glycocholic acid by precipitation from the clarified solution, with turbidity lower than 3 FTU (formazin turbidity unit), of the sodium salt thereof, with solutions of strong acids, such as HCl 1N, up to a pH comprised between 2.0 and 2.5.

A final mixing in water, upon precipitation, changes the crystalline form of the product, making it suitably filterable.

The following examples illustrate the invention, without restricting it in any manner whatsoever.

### EXAMPLES

### Key

GCAEE: glycocholic acid ethyl-ester
CA: cholic acid
GGCAEE glicilglycocholic acid ethyl-ester
GCA: glycocholic acid

### Example 1

### Synthesis of crude GCAEE.

Added into a clean and dry 2-litre glass flask are:
- cholic acid 80.7 g equivalent to 0.197 moles
- acetone 650 ml
- water 19 ml
- triethylamine 20,8 g

It is stirred and the suspension is cooled between 0 and 5° C.

Also prepared in a 1-litre flask is a solution containing:
- glycine ethyl-ester HCl 30 g
- water 545 ml
- triethylamine 21,8 g

When the suspension of cholic acid is between 0 and 5° C, ethyl chloroformate 22,3 g is dripped maintaining the temperature of the suspension between 0 and 5° C. Upon terminating adding ethyl chloroformate, stirring is carried out for 15-20 minutes, between 0 and 5° C. Then the aqueous solution of glycine ethyl-ester is poured onto the suspension of the mixed anhydride of cholic acid and the solution is stirred for about 12 hours. Lastly, the suspension is filtered and the panel is washed using water/acetone 50/50 v/v 60 ml.

A wet yield is obtained of 89 g, i.e. 80g dry equivalent to 82% stoichiometric. TLC purity: CA free by about 1 %; GGCAEE lower or equivalent to 0.25 %. Other correlated impurities: lower than 0.2 % each lower than 0.2 % in total. TLC eluent:
Diisopropylether/isooctane/isopropanol/metanol/formic acid/water 50:20: 15: 12:3: 1

### Example 2

### Purification of crude GCAEE.

Added into a 1-litre glass flask are:
- crude and wet GCAEE 44 g
- acetone / water 50/50 v/v 425 ml

The suspension is stirred and boiled to 65 °C. The suspension is boiled for 30 minutes and then it is cooled to room temperature. It is left under stirring for 12 hours. The suspension is filtered and the panel is washed on the filter with 25 ml of acetone / water 50/50 v/v.

A wet yield is obtained of 33.7 g, i.e. 30.3g dry equivalent to 76.5 w/w with respect to the crude product.

TLC Purity, eluent of the crude product: CA lower than 0.3 %; GGCAEE lower than 0.2 %; other impurities lower than 0.2 in total.

### Example 3

### Hydrolysis and precipitation of GCA

Added into a clean and dry 1-litre glass flask are:
- water 189 ml
- NaOH 1.89 g

Stirring is carried out for about 1 hour in such a manner that all the sodium hydroxide dissolves, at ambient temperature.

Once the solution is complete, 16.9 g of purified wet GCAEE are added and the suspension is stirred for about 12 hours, at a temperature comprised between 20 and 28°C.

Then, the clear solution is taken to a pH comprised between 7.0 and 7.5 with about 50ml of HCl 1N. The aqueous solution is washed twice using ethyl acetate 75 ml, at a temperature between 35 and 40 °C.

Thus, the separated aqueous solution is heated under vacuum to eliminate the ethyl acetate. The aqueous solution thus obtained is diluted with 100 ml of water, and the pure GCA is obtained by heating the solution between 35 and 40 °C and precipitation with HCl 1N, up to a pH comprised between 2.0 and 2.5.

The wet product is dried under vacuum between 50 and 60° C.

The dry yield of the sesquihydrate product is 14 g, equivalent to 92.3% stoichiometric.

### TLC purity

CA lower than 0.4 % - GGCAEE lower than 0.1 % - Other impurities lower than 0.2 % in total. Glycine not observable.

## Claims

1. Process for the preparation of glycocholic acid comprising the following steps:
a) making a mixed anhydride of cholic acid by suspension of cholic acid in acetone or in aqueous acetone in presence of a base, and subsequent reaction with an alkyl chloroformate;
b) aminolysis of the so obtained mixed anhydride of cholic acid with an aqueous solution of a glycine ester, resulting in an ester of glycocholic acid.

2. Process according to claim 1, wherein the base is an amine, preferably a trialkylamine and more preferably triethylamine or tributylamine.

3. Process according to claim 1, wherein the alkyl chloroformate is methyl chloroformate, ethyl chloroformate or propyl chloroformate, preferably ethyl chloroformate.

4. Process according to claim 1, wherein the temperature of the step a) is maintained between -5 °C and +10 °C, preferably between 0 °C and +5 °C.

5. Process according to claim 1, wherein the glycine ester is a C₁-C₄ alkyl ester, preferably glycine ethyl ester.

6. Process according to claim 1, wherein the aminolysis of step b) is performed under stirring, for a time comprised between about 10 and 14 hours, preferably about 12 hours.

7. Process according to claim 1, comprising the hydrolysis of the ester of glycocholic acid by treatment with a strong aqueous base and the precipitation of glycocholic acid by acidification of the correspondent sodium salt.

8. Process according to claim 7, wherein the strong aqueous base is aqueous NaOH or aqueous KOH, preferably aqueous NaOH.

9. Process according to claim 7, wherein the acidification is performed to arrive at a pH comprised between 2.0 and 2.5.

10. Process according to claim 7, wherein the acidification is performed with hydrochloride acid.

## Patentansprüche

1. Verfahren zur Herstellung von Glycocholsäure, umfassend die folgenden Schritte:
a) Anfertigen eines gemischten Cholsäureanhydrids durch Suspendieren von Cholsäure in Aceton oder in wässrigem Aceton in Gegenwart einer Base, und anschließende Reaktion mit einem Alkylchlorformat;
b) Aminolyse des so erhaltenen gemischten Cholsäureanhydrids mit einer wässrigen Lösung eines Glycinesters, was zu einem Glycocholsäureester führt.

2. Verfahren gemäß Anspruch 1, wobei die Base ein Amin, bevorzugt ein Trialkylamin und weiter bevorzugt Triethylamin oder Tributylamin, ist.

3. Verfahren gemäß Anspruch 1, wobei das Alkylchlorformat Methylchlorformat, Ethylchlorformat oder Propylchlorformat, bevorzugt Ethylchlorformat, ist.

4. Verfahren gemäß Anspruch 1, wobei die Temperatur von Schritt a) zwischen -5°C und +10°C, bevorzugt zwischen 0°C und +5°C, gehalten wird.

5. Verfahren gemäß Anspruch 1, wobei der Gycinester ein C₁-C₄-Alkylester, bevorzugt Glycinethylester, ist.

6. Verfahren gemäß Anspruch 1, wobei die Aminolyse von Schritt b) unter Rühren durchgeführt wird, für eine Zeit, die ungefähr zwischen 10 und 14 Stunden liegt, bevorzugt ungefähr 12 Stunden.

7. Verfahren gemäß Anspruch 1, umfassend die Hydrolyse des Glycocholsäureesters durch Behandlung mit einer starken wässrigen Base und die Präzipitation der Glycocholsäure durch Ansäuern des korrespondierenden Natriumsalzes.

8. Verfahren gemäß Anspruch 7, wobei die starke wässrige Base wässriges NaOH oder wässriges KOH, bevorzugt wässriges NaOH, ist.

9. Verfahren gemäß Anspruch 7, wobei die Ansäuerung durchgeführt wird, um bei einem pH anzugelangen, der zwischen 2,0 und 2,5 liegt.

10. Verfahren gemäß Anspruch 7, wobei die Ansäuerung mit Salzsäure durchgeführt wird.

## Revendications

1. Procédé de préparation d'acide glycocholique comprenant les étapes suivantes :
a) préparation d'un anhydride d'acide cholique en mélange par suspension d'acide cholique dans de l'acétone ou une solution aqueuse d'acétone en présence d'une base, puis par réaction avec un chloroformiate d'alkyle;
b) aminolyse de l'anhydride d'acide cholique en mélange ainsi obtenu avec une solution aqueuse d'un ester de glycine, donnant un ester d'acide glycocholique.

2. Procédé selon la revendication 1, dans lequel la base est une amine, de préférence une trialkylamine et plus préférablement la triéthylamine ou la tributylamine.

3. Procédé selon la revendication 1, dans lequel le chloroformiate d'alkyle est le chloroformiate de méthyle, le chloroformiate d'éthyle ou le chloroformiate de propyle, de préférence le chloroformiate d'éthyle.

4. Procédé selon la revendication 1, dans lequel la température de l'étape a) est maintenue entre -5 °C. et +10 °C, de préférence entre 0 °C et +5 °C.

5. Procédé selon la revendication 1, dans lequel l'ester de glycine est un ester alkylique en C₁ à C₄, de préférence l'ester éthylique de glycine.

6. Procédé selon la revendication 1, dans lequel l'aminolyse de l'étape b) est réalisée sous agitation, pendant une période comprise entre environ 10 et 14 heures, de préférence d'environ 12 heures.

7. Procédé selon la revendication 1, comprenant l'hydrolyse de l'ester d'acide glycocholique par traitement avec une solution aqueuse d'une base forte et la précipitation de l'acide glycocholique par acidification du sel sodique correspondent.

8. Procédé selon la revendication 7, dans lequel la solution aqueuse de base forte est une solution aqueuse de NaOH ou une solution aqueuse de KOH, de préférence une solution aqueuse de NaOH.

9. Procédé selon la revendication 7, dans lequel l'acidification est réalisée afin d'atteindre un pH compris entre 2,0 et 2,5.

10. Procédé selon la revendication 7, dans lequel l'acidification est réalisée avec de l'acide chlorhydrique.
